(19) Europäisches Patentamt / European Patent Office / Office européen des brevets

(11) **EP 4 501 233 B1**

(12) **EUROPEAN PATENT SPECIFICATION**

(45) Date of publication and mention of the grant of the patent:
**17.12.2025 Bulletin 2025/51**

(21) Application number: **23382800.3**

(22) Date of filing: **31.07.2023**

(51) International Patent Classification (IPC):
$A61B\ 5/282^{(2021.01)}$  $A61B\ 5/332^{(2021.01)}$
$A61B\ 5/367^{(2021.01)}$  $A61B\ 5/00^{(2006.01)}$

(52) Cooperative Patent Classification (CPC):
**A61B 5/6805; A61B 5/282; A61B 5/332; A61B 5/367; A61B 5/745**

(54) **SYSTEM AND METHOD OF 360º THREE-DIMENSIONAL STEREOSCOPIC HOLOGRAPHIC VISUALIZATION FOR THE ELECTRICAL SIGNALS OF THE HEART**

SYSTEM UND VERFAHREN ZUR DREIDIMENSIONALEN STEREOSKOPISCHEN HOLOGRAPHISCHEN 360 °-VISUALISIERUNG FÜR DIE ELEKTRISCHEN SIGNALE DES HERZENS

SYSTÈME ET PROCÉDÉ DE VISUALISATION HOLOGRAPHIQUE STÉRÉOSCOPIQUE TRIDIMENSIONNELLE À 360 ? POUR LES SIGNAUX ÉLECTRIQUES DU C UR

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**05.02.2025 Bulletin 2025/06**

(73) Proprietor: **Spika Tech, S.L.**
**28018 Madrid (ES)**

(72) Inventors:
- **ARNAIZ LOZANO, María Esther**
  **28018 Madrid (ES)**
- **ZÚÑIGA ARNAIZ, Alicia**
  **28018 Madrid (ES)**
- **ZÚÑIGA ARNAIZ, Cristina**
  **28018 Madrid (ES)**

(74) Representative: **Ungria López, Javier**
**Avda. Ramón y Cajal, 78**
**28043 Madrid (ES)**

(56) References cited:
**CN-U- 204 207 739     US-A1- 2003 120 163**

- **SALINET JOÃO ET AL:** "Electrocardiographic Imaging for Atrial Fibrillation: A Perspective From Computer Models and Animal Experiments to Clinical Value", FRONTIERS IN PHYSIOLOGY, vol. 12, 30 April 2021 (2021-04-30), XP93104464, DOI: 10.3389/fphys.2021.653013

- **KALININ ALEXANDER ET AL:** "Solving the Inverse Problem of Electrocardiography on the Endocardium Using a Single Layer Source", FRONTIERS IN PHYSIOLOGY, vol. 10, 5 February 2019 (2019-02-05), XP093104475, DOI: 10.3389/fphys.2019.00058

- **BEAR LAURA R ET AL:** "The Impact of Torso Signal Processing on Noninvasive Electrocardiographic Imaging Reconstructions", IEEE TRANSACTIONS ON BIOMEDICAL ENGINEERING, IEEE, USA, vol. 68, no. 2, 18 June 2020 (2020-06-18), pages 436 - 447, XP011832147, ISSN: 0018-9294, [retrieved on 20210120], DOI: 10.1109/TBME.2020.3003465

- **COLL-FONT JAUME ET AL:** "Tracking the Position of the Heart From Body Surface Potential Maps and Electrograms", FRONTIERS IN PHYSIOLOGY, vol. 9, 3 December 2018 (2018-12-03), XP093104519, DOI: 10.3389/fphys.2018.01727

- **OLIVER ZETTINIG ET AL:** "Data-driven estimation of cardiac electrical diffusivity from 12-lead ECG signals", MEDICAL IMAGE ANALYSIS, vol. 18, no. 8, 1 December 2014 (2014-12-01), pages 1361 - 1376, XP55202563, ISSN: 1361-8415, DOI: 10.1016/j.media.2014.04.011

**Description**

**OBJECT OF THE INVENTION**

The present invention relates to a system and method of 360° three-dimensional stereoscopic holographic visualization for the electrical signals of the heart. The present invention describes a non-invasive cardiac navigation system, designed to visualize the electrical signals of the heart on a 360° three-dimensional stereoscopic holographic display. The system uses a textile data acquisition device highly adaptable to the patient's torso, made up of electrodes that record their electrical signals. From the signals collected from the torso, the original electrical signals of the heart are inferred by the solution proposed in the present invention for the inverse problem of electrophysiology.

The main advantage of the present invention lies in its non-invasive nature since it does not require the introduction of catheters into the patient's body to capture the electrical signals. In addition, the ability to visualize signals in three dimensions via a 360° stereoscopic holographic display facilitates better understanding and analysis of heart signals. The present invention can be deployed in a cloud computing service, which facilitates real-time signal processing.

**BACKGROUND OF THE INVENTION AND TECHNICAL PROBLEM TO BE RESOLVED**

The evolution of cardiology has been marked by the development of increasingly precise and less invasive diagnostic and treatment techniques. From the invention of the electrocardiograph in the 19th century to the advent of non-invasive imaging techniques such as echocardiography, magnetic resonance imaging, and computed tomography, medicine has experienced significant advances in the diagnosis and treatment of heart disease.

However, despite these advances, limitations remain in the ability to intuitively and accurately visualize and understand the electrical signals of the heart. To a large extent, this is because most current diagnostic techniques employ invasive catheters that are inserted directly into the user/patient's body.

Since the diagnosis and treatment of heart disease is essential, it is important to note that focal arrhythmias and atrial fibrillation are two of the most common and potentially dangerous heart conditions. Focal arrhythmias originate from a specific point on the heart due to abnormal electrical activity, while atrial fibrillation is an arrhythmia characterized by chaotic and disorganized electrical activity, which can lead to decreased heart function and increased risk complications such as stroke or heart failure.

In this context, the need arises for a non-invasive cardiac navigation solution such as the present invention, which makes it possible to visualize the electrical signals of the heart in a three-dimensional manner and in real time, without the need to introduce electrodes into the patient's body. The ability to visualize the electrical signals of the heart in this way would facilitate a better understanding and analysis of the electrical activity of the heart, which could lead to improvements in the diagnosis and treatment of heart disease. SALINET JOÃO ET AL: "Electrocardiographic Imaging for Atrial Fibrillation: A Perspective From Computer Models and Animal Experiments to Clinical Value", FRONTIERS IN PHYSIOLOGY, vol. 12, 30 April 2021 (2021-04-30), DOI: 10.3389/fphys.2021.653013 describes the visualization of cardiac activity.

The ability to visualize the heart's electrical signals in a 360°, three-dimensional, stereoscopic, holographic fashion provided by the present invention could be especially useful in the diagnosis and treatment of these cardiac conditions. By allowing a better understanding of the electrical activity of the heart and a more precise identification of the points of origin of focal arrhythmias or the chaotic electrical activity of atrial fibrillation, the present invention contributes to improving the accuracy and efficacy of the diagnosis of various heart conditions.

**DESCRIPTION OF THE INVENTION**

A first aspect of the invention discloses a method of 360° three-dimensional stereoscopic holographic visualization for the electrical signals of the heart. The method comprises:

- preprocessing signals formed by potentials "$V_T$" of a user's torso; where the potentials "$V_T$" are measured by a textile device with electrodes;
- calculating a transfer matrix "$A_{TH}$" between a 3D mesh of the user's heart and a 3D mesh of the user's torso: $V_T = A_{TH} V_H$;
- estimating potentials "$V_H$" of the user's heart from the 3D mesh of the user's heart and the potentials "$V_T$" of the user's torso, where the estimated heart potentials are called $E\left[V_H^i\right]$, for which a value of the parameter $\lambda$ is calculated, which minimizes the expression:

$$\epsilon = ||A_{TH} E[V_H^i] - V_T||^2 + \lambda^2 ||R\, E[V_H^i]||^2$$

where $R$ is the so-called regularization matrix, and the identity matrix has been used; this is equivalent to a zero-order Tikhonov regularization; therefore, $R$ is a square matrix, which has $N_H$ rows and $N_H$ columns;

- recalculating position and orientation of the 3D mesh of the heart, where the position and orientation of the 3D mesh of the heart is defined by the vector $\psi = (x_i, y_i, z_i, \theta_i, \rho_i, \phi_i)$;

- estimating torso potentials for said position: $E[V_T^i] = A_{TH}\, E[V_H^i]$ by calculating the value of $\psi$ which minimizes the following expression:

$$\mathrm{argmin}_\psi ||A\, E[V_H^i] - V_T||^2 + \lambda^2 ||R\, E[V_H^i]||^2$$

- repeating the above steps until $||E[V_T] - V_T|| < \delta$, where $\delta$ is the tolerance that is determined empirically a priori and determines that the algorithm has converged, that is, $\delta$ is a tolerance with a predefined value;
- providing a 360° 3D stereoscopic holographic representation using the 3D visualization means, the estimated heart potentials $E[V_H^i]$ and the 3D mesh of the user's heart.

In one embodiment of the invention, the step of calculating a transfer matrix "$A_{TH}$" between a 3D mesh of the user's heart and a 3D mesh of the user's torso, further comprises: selecting the 3D mesh of the user's heart between a standard 3D mesh and a 3D mesh of the user from computed tomography or magnetic resonance images; and scanning the surface of the user's torso using a device configured for generating 3D meshes.

In one embodiment of the invention, the step of calculating a transfer matrix "$A_{TH}$" further comprises the following sub-steps:

- applying Green's second identity that relates the calculation of electric potentials inside a volume "$\theta$" with said calculation on its surface "S" to the torso and heart 3D meshes, generating the following equation:

$$\int_S \left(V\nabla \frac{1}{r} - \frac{1}{r}\nabla V\right) \cdot \mathbf{n}\, dS = \int_\Theta \left(V\nabla^2 \left(\frac{1}{r}\right) - \frac{1}{r}\nabla^2 V\right) d\Theta, \text{ (E2)}$$

where V is the electric potential and r the distance from an observation point inside the volume between the surface of the torso and the surface of the heart (the integral on the left is over the surface, while the integral on the right is over the volume $\Theta$); the vector n is the normal to the surface element $dS$ and V is the gradient operator;
- applying the following boundary conditions to Green's second identity:

    o C1: $V = V_T$ when the observation point is on the torso and $V = V_H$ when the observation point is on the surface of the heart;
    ○ C2: $\nabla^2 V = 0$; since there are no electrical sources inside the volume between the surface of the heart and the surface of the torso since the active electrical sources are in the heart;
    ○ C3: $\nabla V_T \cdot \mathbf{n} = 0$; there is no electrical current outside the outer surface, so $\nabla V_T \cdot \mathbf{n} = 0$ on the surface of the torso.

In one embodiment of the invention, the step of preprocessing potentials "$V_T$" of a user's torso comprises the following sub-steps:

- identifying and eliminating outliers of the signals formed by the potentials "$V_T$" by considering the potentials "$V_T$" that do not exceed a certain threshold by a given number of standard deviations; this elimination allows to obtain a cleaner and more precise signal;
- applying a "wavelet" transform to decompose the signals formed by the potentials "$V_T$" at different resolution levels; this allows noise to be eliminated without losing relevant information;
- eliminating the wavelet coefficients associated with noise frequencies;
- reconstructing the signals formed by the potentials "$V_T$" using only the remaining coefficients;
- correcting the linear trend of the signals formed by the potentials "$V_T$" by detecting the intervals of the peaks "R" in the resulting electrocardiogram "ECG", the peaks "R" being the local maxima of the QRS complex, which in turn are detected through the inclination of the absolute gradient of the ECG signal;
- selecting at least the ten best seconds of the signals formed by the potentials "$V_T$", using an evaluation of the quality of each signal by means of a relative index. This index is obtained by estimating as most appropriate those values that are most similar to the average sample.

A second aspect of the invention discloses a system of 360° three-dimensional stereoscopic holographic visualization for the electrical signals of the heart. The system comprises:

- a textile device with electrodes, which is adjustable to a user's torso, where the textile device is configured for measuring potentials "$V_T$" of the user's torso by means of the electrodes of the textile device;
- a 3D meshing device, which is configured for creating a 3D mesh of the user's torso;
- a processor that receives the 3D mesh and the potentials of the user's torso, and a 3D mesh of a heart;
- 3D visualization means;

where the processor is configured for:

- preprocessing the potentials of the user's torso: "$V_T$";
- calculating a transfer matrix "$A_{TH}$" between the 3D mesh of the heart and the 3D mesh of the torso: $V_T = A_{TH} V_H$;
- estimating potentials "$V_H$" of the user's heart from the 3D mesh and the potentials "$V_T$" of the user's torso, where the estimated heart potentials are called $E\left[V_H^i\right]$, for which a parameter "$\lambda$" is sought, which minimizes the expression:

$$\epsilon = ||A_{TH}E\left[V_H^i\right] - V_T||^2 + \lambda^2||RE\left[V_H^i\right]||^2;$$

  where R is the so-called regularization matrix, and the identity matrix has been used. This is equivalent to a zero-order Tikhonov regularization. Therefore, $R$ is a square matrix, which has $N_H$ rows and $N_H$ columns;
- recalculating position and orientation of the 3D mesh of the heart, where the position and orientation of the 3D mesh of the heart is defined by the vector $\psi = (x_i, y_i, z_i, \theta_i, \rho_i, \phi_i)$;

- estimating torso potentials: $E\left[V_T^i\right] = A_{TH}\ E\left[V_H^i\right]$, by calculating the value of $\psi$ which minimizes the following expression:

$$\mathrm{argmin}_\psi||AE\left[V_H^i\right] - E\left[V_T^i\right]||^2 + \lambda^2||RE\left[V_H^i\right]||^2$$

- repeating the above steps until $||E[V_T] - V_T|| < \delta$, where $\delta$ is the tolerance that is determined empirically a priori and determines that the algorithm has converged, that is, $\delta$ is a tolerance with a predefined value;
- providing a 360° 3D stereoscopic holographic representation using the 3D visualization means, the estimated heart potentials $E\left[V_H^i\right]$ and the 3D mesh of the user's heart.

In one embodiment of the invention, the visualization means are selected from a virtual reality viewer and a holographic display.

**BRIEF DESCRIPTION OF THE FIGURES**

To complete the description of the invention and for the purpose of helping to better understand its features according to a preferred embodiment thereof, a set of drawings is attached in which the following figures are depicted in an illustrative and non-limiting manner:

Figure 1 represents a diagram showing the system of 360° three-dimensional stereoscopic holographic visualization for the electrical signals of the heart of the present invention.
Figure 2 represents a flowchart of the method of the present invention from the pre-processing of the signals with the potentials of the user's torso to the final visualization, through the reconstruction of the inverse electrocardiography problem together with the optimization of the position and orientation of the heart.

A list of the references used in the figures is provided below:

1.- Not used;

2.- Textile device; 2': electrodes of the textile device; 2" wireless communication means (WiFi, Bluetooth) of the textile device;

3.- User;

4.- processor (local); 4' cloud processor;

5.- Not used;

6.- 3D meshing device;

7.- External source of computed tomography or magnetic resonance images;

8.- Preprocessing of the signals with the potentials "$V_T$" of the user's torso;

9.- Generation of 3D meshes of the torso and heart;

10.- Virtual reality viewer;

11.- Holographic display;

12.- Computer; 12'. Computer;

13 to 19.- Not used;

20 to 31: method steps.

## DESCRIPTION OF A PREFERRED EMBODIMENT OF THE INVENTION

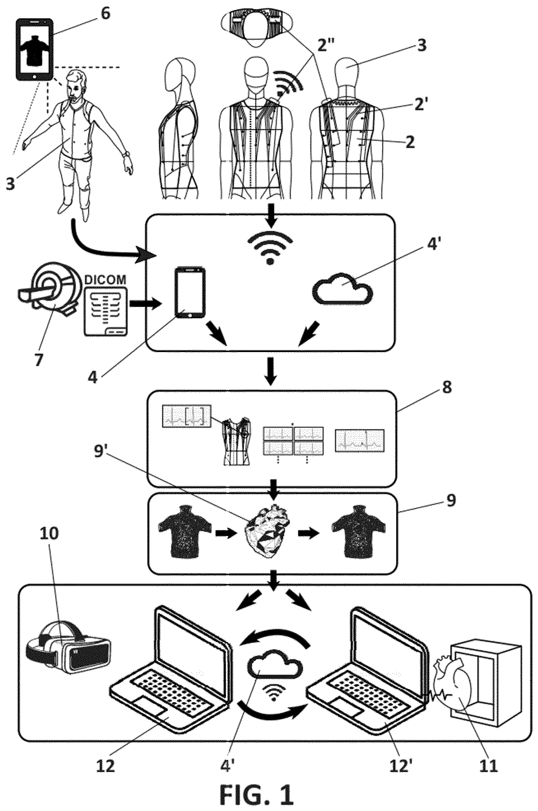

Figure 1 represents a diagram showing the system of 360° three-dimensional stereoscopic holographic visualization for the electrical signals of the heart. The system comprises the textile device 2 in the form of a vest. The textile device 2 has thirty electrodes 2' distributed throughout same and is adjustable to the user's torso 3. The electrodes 2' of the textile device 2 measure the potentials "$V_T$" of the torso. The electrodes 2' of the textile device 2 can be dry embroidered electrodes that improve the measurement of potential signals. The textile device 2 has the wireless communication means 2" to connect with the processor 4 that can be a local processor 4 or a cloud processor 4'. The system also comprises the 3D meshing device 6, which is configured for creating a 3D mesh of the user's torso. The 3D meshing device 6 may be a smartphone with an advanced video camera that captures the surface of the user's torso and recreates a three-dimensional mesh using an algorithm based on artificial intelligence. The system also comprises processor 4, which in Figure 1 is shown as part of a smart mobile device, which receives the 3D mesh created by the 3D meshing device 6 and the signals with potentials of the user's torso from the textile device 2. The processor 4 needs a 3D mesh of a heart. For this, the processor can have a 3D mesh stored by default or it can receive a 3D mesh from the user from computed tomography or magnetic resonance images from an external source 7. The processor 4,4' is configured for preprocessing the signals with the potentials "$V_T$" of the user's torso (8 in Figure 1) and reconstructing the inverse electrocardiography problem together with optimization of the position and orientation of the heart. That is, the processor 4,4' (9 in Figure 1) is thus configured for estimating the electrical potentials of the heart, generating a 3D mesh with the shape of the user's torso 9" and the potentials distributed by the 3D mesh of the user's torso measured by the textile device 2, and generating a 3D mesh combining the 3D mesh of the heart and the 3D mesh of the user's torso. Finally, the system includes the 3D visualization means which can be the computer 12 communicated with the virtual reality viewer 10, and/or the computer 12' communicated with the holographic screen 11.

The system of the present invention has a multi-user connection to share a virtual workspace and synchronize devices with different methods of visualization.

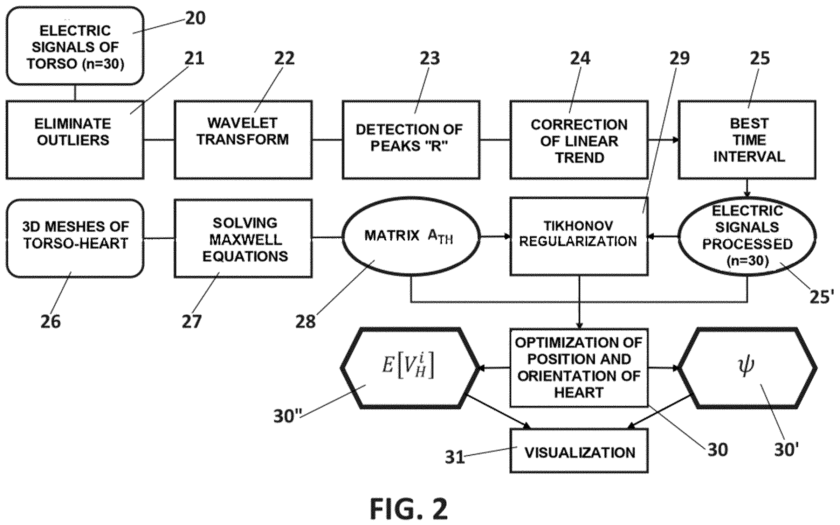

Figure 2 discloses the method of the present invention which is carried out by the processor of the system of the present invention. That is, Figure 2 shows how the pre-processing of the signals with the potentials "$V_T$" of the user's torso (8 in Figure 1) and reconstruction of the inverse electrocardiography problem together with optimization of the position and orientation of the heart are carried out.

The pre-processing of the signals with the potentials "$V_T$" of the user's torso includes detection and elimination of outliers, noise removal, linear trend correction, and selection of the best time interval for processed signals. Therefore, the method comprises the following sub-steps:

a) firstly, using the signals formed by potentials "$V_T$" (20) and measurements in the user's torso through the electrodes of the textile device, the outliers present in the torso signals are identified and eliminated (21); for this purpose, those potential values that do not exceed a predetermined threshold by a given number of standard deviations are considered; this elimination allows a cleaner and more precise signal to be obtained;

b) subsequently, a *wavelet* transform (22) is applied to decompose the signal at different resolution levels, which makes it possible to eliminate noise without losing relevant information; after eliminating the *wavelet* coefficients associated with noise frequencies, the signal is reconstructed using only the remaining coefficients;

c) furthermore, the linear trend (24) of the signal is corrected by detecting (23) the intervals of the peaks R in the resulting electrocardiogram (ECG); peaks R are identified as local peaks of the QRS complex, which in turn is detected through the inclination of the absolute gradient of the ECG signal;

d) lastly, at least the ten best seconds of the processed signals are selected (25), using an evaluation of the quality of each signal by means of a relative index; this index is obtained by estimating as most appropriate those values that are most similar to the average sample. As a result, the signals formed by processed potentials "$V_T$" (25') that can be written mathematically as the matrix $V_T$.

Continuing with Figure 2, the method comprises the following steps regarding the reconstruction of the inverse electrocardiography problem.

The inverse electrocardiography problem consists of estimating the electrical activity of the heart from data on the electrical activity of the torso. Mathematically, this can be written as:

$$V_T = A_{TH}V_H. \qquad (E1)$$

The data matrix $V_T$ consists of the potentials in the torso that are measured by the textile device. The potentials $V_H$ in the heart are unknown and are the ones to be estimated, where $A_{TH}$ it is the so-called transfer matrix between three-dimensional meshes of heart to torso.

To solve the inverse problem, the boundary element method (BEM) is used. With this method and the triangular 3D meshes (26) of the borders of the starting geometries, that is, the geometry of the torso and the geometry of the heart inside, the BEM method allows developing (27) Maxwell equations for the electric field. By applying Green's second identity that relates the calculation of electrical potentials inside a volume "θ" with said calculation on its surface "S", the following equation is obtained:

$$\int_S \left( V\nabla \frac{1}{r} - \frac{1}{r}\nabla V \right) \cdot \mathbf{n}\, dS = \int_\Theta \left( V\nabla^2 \left( \frac{1}{r} \right) - \frac{1}{r}\nabla^2 V \right) d\Theta, \qquad (E2)$$

where "$V$" is the electric potential and "r" the distance from an observation point inside the volume between the surface of the torso and the surface of the heart. The integral on the left is over the surface "$S$", while the integral on the right is over the volume "$\Theta$". The vector "$\mathbf{n}$" is the normal to the surface element $dS$ and V is the gradient operator.

The three-dimensional mesh of the user's torso has been pre-generated, and the 3D mesh of the user's heart can either be a 3D mesh of a predetermined standard heart or a 3D mesh (model) of the user's heart is reconstructed from medical imaging, for example, CT or MRI.

These equations can be discretized using this triangular meshing in multiple equations ($N_T$), one for each node of the torso. These are solved with the help of the following conditions:

- $V = V_T$ when the observation point is on the torso and $V = V_H$ when the observation point is on the surface of the heart (C1);
- there are no electrical sources inside the volume between the two surfaces since the active electrical sources are in the heart. Therefore, $\nabla^2 V = 0$ (C2);
- there is no electrical current outside the outer surface, so $\nabla V_T \cdot \mathbf{n} = 0$ on the surface of the torso (C3).

Equation (E1) can be solved using the previous conditions and Green's identity (E2), and decomposing it in parts, so that a solution for the matrix $A_{TH}$ is obtained.

Starting from the identity of (E2) and with the conditions (C2, C3) the potential is obtained as a function of x:

$$V(x) = \frac{1}{4\pi}\int_S \frac{V r \cdot n}{r^2}\, dS + \frac{1}{4\pi}\int_S \frac{\nabla V \cdot n}{r}\, dS, \quad (E3)$$

where $r$ is a unit vector in the direction of $r$, i.e., from the observation point $x$. By separating the surface $S$ into $S_T$ and $S_H$, and using condition (C3), the following is arrived at:

$$V(x) = -\frac{1}{4\pi}\int_{S_H} \frac{V_H r \cdot n}{r^2}\, dS_H + \frac{1}{4\pi}\int_{S_T} \frac{V_T r \cdot n}{r^2}\, dS_T - \frac{1}{4\pi}\int_{S_H} \frac{\nabla V_H \cdot n}{r}\, dS_H. \quad (E4)$$

It can be solved (E4) by developing at observation points $x$ very close to the mesh of the torso or the mesh of the heart and using condition (C1), such that:

$$-V_T^x + \frac{1}{4\pi}\int_{S_T} V_T\, d\Omega_{TT}^x - \frac{1}{4\pi}\int_{S_H} V_H\, d\Omega_{TH}^x - \frac{1}{4\pi}\int_{S_H} \frac{\nabla V_H \cdot n}{r^x}\, dS_H = 0, \quad (E5)$$

$$\frac{1}{4\pi}\int_{S_T} V_T d\Omega^x_{HT} - V^x_H - \frac{1}{4\pi}\int_{S_H} V_H d\Omega^x_{HH} - \frac{1}{4\pi}\int_{S_H} \frac{\nabla V_H \cdot \boldsymbol{n}}{r^x} dS_H = 0, \quad (E6)$$

where $d\Omega$ is the solid angle element from the point x. Each of the elements of (E5) can be written as:

$$-V^x_T + \frac{1}{4\pi}\int_{S_T} V_T d\Omega^x_{TT} = \sum_{j=1}^{N_T} p^{ij}_{TT} V^j_B, \quad (E7)$$

$$-\frac{1}{4\pi}\int_{S_H} V_H d\Omega^x_{TH} = \sum_{j=1}^{N_H} p^{ij}_{TH} V^j_H, \quad (E8)$$

$$-\frac{1}{4\pi}\int_{S_H} \frac{\nabla V_H \cdot \boldsymbol{n}}{r^x} dS_H = \sum_{j=1}^{N_H} g^{ij}_{TH} \Gamma^j_H, \quad (E9)$$

And, similarly, those of (E6) are developed as follows:

$$\frac{1}{4\pi}\int_{S_T} V_T d\Omega^x_{HT} = \sum_{j=1}^{N_H} p^{ij}_{HT} V^j_T, \quad (E10)$$

$$-V^x_H - \frac{1}{4\pi}\int_{S_H} V_H d\Omega^x_{HH} = \sum_{j=1}^{N_H} p^{ij}_{HH} V^j_H, \quad (E11)$$

$$-\frac{1}{4\pi}\int_{S_H} \frac{\nabla V_H \cdot n}{r^x} dS_H = \sum_{j=1}^{N_H} g^{ij}_{HH} \Gamma^j_H, \quad (E12)$$

where $V_T$ and $V_H$ are vectors with the potentials at the $N_T$ and $N_H$ positions of the nodes of the mesh of the torso and heart and $\Gamma_H$ the normal components of the gradients in the heart.

In this way, equations (E5) and (E6) can be rewritten as follows:

$$p^j_{TT} V_T + p^j_{TH} V_H + g^j_{TH} \Gamma_H = 0, (E13)$$

$$p^j_{HT} V_T + p^j_{HH} V_H + g^j_{HH} \Gamma_H = 0, \quad (E14)$$

which in matrix form are represented as two systems of equations:

$$P_{TT} V_T + P_{TH} V_H + G_{TH} \Gamma_H = 0, (E15)$$

$$P_{HT} V_T + P_{HH} V_H + G_{HH} \Gamma_H = 0. (E16)$$

The first system (E15) has $N_T$ equations that correspond to equations in each node of the mesh of the torso, while the second system (E16) has $N_H$, corresponding to each node or vertex of the surface of the heart.

The gradients can be solved by using:

$$\Gamma_H = -G^{-1}_{HH}(P_{HT} V_T + P_{HH} V_H). \quad (E17)$$

In this way, the transfer matrix $A_{TH}$ (28) is arrived at and can be written as:

$$A_{TH} = \left(P_{TT} - G_{TH} G^{-1}_{HH} P_{HT}\right)^{-1}\left(G_{TH} G^{-1}_{HH} P_{HH} - P_{TH}\right). \quad (E18)$$

However, to obtain the potentials in the heart, the matrix $A_{TH}$ must be inverted, for which purpose regularization techniques are used since the inverse problem is not well defined. A mathematical problem is well defined if its solution exists, if the solution is unique, and if the solution is stable. This is not the case in the inverse problems since the noise in the signals or the possible errors in the reconstruction of the mesh can lead to very different solutions.

To invert the matrix $A_{TH}$, Tikhonov regularization (29) is used, in which a value of the parameter $\lambda$ is sought, which minimizes the expression:

$$\epsilon = ||A_{TH}V_H - V_T||^2 + \lambda^2||RV_H||^2. \qquad (E19)$$

Matrix $R$ is the so-called regularization matrix, and the identity matrix (zero-order Tikhonov), which is a square matrix, has been used. In this case, the regularization matrix has $N_H$ rows and $N_H$ columns. The parameter $\lambda$ describes a balance between the prediction and the measured values and for its choice the *"L-curve"* method, which seeks a balance between the two terms of the expression to be minimized, the norm $||AV_H - V_T||^2$ and $||RV_H||^2$, is used.

Continuing with Figure 2, regarding the optimization of the position and orientation of the heart, the method comprises the following steps that are detailed below.

The optimization of the position and orientation of the heart (30) consists of, in an iterative manner, optimizing the position of the heart by minimizing the error in the estimated potentials of the torso. Until now, the meshing of the fixed heart had been considered to obtain the coefficients $A_{TH}$. It must be taken into account that the coefficients depend on the distances between the nodes of the 3D mesh of the torso and the nodes of the 3D mesh of the heart. If the heart is reoriented, the matrix $A_{TH}$ must be recalculated. In this second part, a vector $\psi = (x, y, z, \theta, \rho, \phi)$ which denotes the position and orientation of the heart in a three-dimensional space, with three axes of rotation, with respect to the horizontal, vertical and lateral axes, is sought.

The steps to follow to find the optimal position and orientation follow the flow indicated below:

1. Choose a position and rotation of the heart, given by six variables $\psi = (x_i, y_i, z_i, \theta_i, \rho_i, \phi_i)$ (30');
2. Calculate the matrix $A_{TH}$ for the current position and orientation depending on the vector $\psi$;

3. Solve the inverse problem as described above, to obtain estimated heart potentials $E\left[V_H^i\right]$ (30");

4. Solve the direct problem using $V_T = AV_H$ (E1), obtaining estimated torso potentials $E\left[V_T^i\right]$;

5. Repeat the above steps until the algorithm converges by the condition of stopping $||E[V_T] - V_T|| < \delta$, where $\delta$ is the tolerance. This tolerance is determined empirically a priori and establishes when the algorithm has converged.

Finally, the method of the present invention comprises the visualization (31), in a virtual reality viewer or on a holographic display, of the estimated heart potentials $E\left[V_H^i\right]$, the 3D mesh of the user's heart, the measured potentials of the torso and/or the 3D mesh of the torso, individually or in combination.

**Claims**

1. A method of 360° three-dimensional stereoscopic holographic visualization for the electrical signals of the heart, said method comprises:

   • preprocessing (20-25) signals formed by potentials "$V_T$" of a user's torso; where the potentials "$V_T$" are measured by a textile device with electrodes;
   • calculating (26-28) a transfer matrix "$A_{TH}$" between a 3D mesh of the user's heart and a 3D mesh of the user's torso: $V_T = A_{TH} V_H$;
   • estimating (29) potentials "$V_H$" of the user's heart from the 3D mesh of the user's heart and the potentials "$V_T$" of the user's torso, where the estimated heart potentials are called $E\left[V_H^i\right]$, for which a value of the parameter "$\lambda$" is calculated, which minimizes the expression:

   $$\epsilon = ||A_{TH}E\left[V_H^i\right] - V_T||^2 + \lambda^2||R\,E\left[V_H^i\right]||^2$$

   where $R$ is an identity matrix, which is called a regularization matrix; such that $R$ is a matrix of $N_H$ rows and $N_H$ columns;
   • recalculating (30) position and orientation of the 3D mesh of the heart, where the position and orientation of the 3D mesh of the heart is defined by the vector $\psi = (x_i, y_i, z_i, \theta_i, \rho_i, \phi_i)$;
   • estimating (30) torso potentials: $E\left[V_T^i\right] = A_{TH}\,E\left[V_H^i\right]$ by calculating the value of $\psi$ which minimizes the

following expression:

$$\text{argmin}_{\psi}||AE[V_H^i] - V_T||^2 + \lambda^2||RE[V_H^i]||^2;$$

- repeating (30) the previous steps until $||E[V_T] - V_T|| < \delta$, where $\delta$ is a tolerance with a predefined value;
- providing (31) a 360° 3D stereoscopic holographic representation using the 3D visualization means, the estimated heart potentials $E[V_H^i]$ and the 3D mesh of the user's heart.

2. The method of claim 1, **characterized in that** the step of calculating a transfer matrix "$A_{TH}$" between a 3D mesh of the user's heart and a 3D mesh of the user's torso, further comprises:

- selecting (26) the 3D mesh of the user's heart from a standard 3D mesh and a 3D mesh of the user from images selected from computed tomography images and magnetic resonance images;
- scanning (26) a surface of the user's torso using a device configured for generating 3D meshes.

3. The method of claim 1, **characterized in that** the step of calculating a transfer matrix "$A_{TH}$", further comprises the following sub-steps:

- applying Green's second identity that relates the calculation of electric potentials inside a volume "$\theta$" with said calculation on its surface "S" to the torso and heart 3D meshes, generating the following equation:

$$\int_S \left(V\nabla\frac{1}{r} - \frac{1}{r}\nabla V\right) \cdot \mathbf{n}\,dS = \int_\Theta \left(V\nabla^2\left(\frac{1}{r}\right) - \frac{1}{r}\nabla^2 V\right)d\Theta, \text{ (E2)}$$

where $V$ is the electric potential and r the distance from an observation point inside the volume between the surface of the torso and the surface of the heart; the vector n is the normal to the surface element $dS$ and V is the gradient operator;
- applying the following boundary conditions to Green's second identity:

  ∘ C1: $V = V_T$ when the observation point is on the torso and $V = V_H$ when the observation point is on the surface of the heart;
  ∘ C2: $\nabla^2 V = 0$;
  ∘ C3: $\nabla V_T \cdot \boldsymbol{n} = 0$.

4. The method of claim 1, **characterized in that** the step of preprocessing potentials "$V_T$" of a user's torso comprises the following sub-steps:

- identifying and eliminating (21) outliers of the signals formed by the potentials "$V_T$" by considering the potentials "$V_T$" that do not exceed a certain threshold by a given number of deviations;
- applying (22) a "wavelet" transform to decompose the signals formed by the potentials "$V_T$" at different resolution levels;
- eliminating (22) the wavelet coefficients associated with noise frequencies;
- reconstructing (22) the signals formed by the potentials "$V_T$" using only the remaining coefficients;
- correcting (24) the linear trend of the signals formed by the potentials "$V_T$" by detecting (23) the intervals of the peaks "R" in the resulting electrocardiogram "ECG", the peaks "R" being the local maxima of the QRS complex, which in turn are detected through the inclination of the absolute gradient of the ECG signal;
- selecting (25) at least the ten best seconds of the signals formed by the potentials "$V_T$", using an evaluation of the quality of each signal by means of a relative index.

5. A system of 360° three-dimensional stereoscopic holographic visualization for the electrical signals of the heart, said system comprising:

- a textile device (2) with electrodes (2'), which is adjustable to a user's torso (3), where the textile device is configured for measuring potentials "$V_T$" of the user's torso by means of the electrodes of the textile device;
- a 3D meshing device (6), which is configured for creating a 3D mesh of the user's torso (3);
- a processor (4,4') that receives the 3D mesh and potentials of the user's torso, and a 3D mesh of a heart;

• 3D visualization means (10,11);

where the processor (4,4') is configured for:

• preprocessing the potentials of the user's torso: "$V_T$";
• calculating a transfer matrix "$A_{TH}$" between the 3D mesh of the heart and the 3D mesh of the torso: $V_T = A_{TH} V_H$;
• estimating $E\left[V_H^i\right]$ potentials "$V_H$" of the user's heart from the 3D mesh and the potentials "$V_T$" of the user's torso, where the estimated heart potentials are called $E\left[V_H^i\right]$ for which a parameter "$\lambda$" is sought, which minimizes the expression:

$$\epsilon = ||A_{TH}E\left[V_H^i\right] - V_T||^2 + \lambda^2||RE\left[V_H^i\right]||^2;$$

where R is an identity matrix, which is called a regularization matrix; such that R is a matrix of $N_H$ rows and $N_H$ columns;
• recalculating position and orientation of the 3D mesh of the heart, where the position and orientation of the 3D mesh of the heart is defined by the vector $\psi = (x_i, y_i, z_i, \theta_i, \rho_i, \phi_i)$;

• estimating torso potentials: $E\left[V_T^i\right] = A_{TH} E\left[V_H^i\right]$ by calculating the value of $\psi$ which minimizes the following expression:

$$\mathrm{argmin}_\psi ||AE\left[V_H^{\bar{i}}\right] - \dot{E}\left[V_T^i\right]||^2 + \lambda^2||RE\left[V_H^i\right]||^2;$$

• repeating the above steps until $||E[V_T] - V_T|| < \delta$, where $\delta$ is the tolerance that is determined empirically a priori and determines that the algorithm has converged, that is, $\delta$ is a tolerance with a predefined value;
• providing a 360° 3D stereoscopic holographic representation using the 3D visualization means, the estimated heart potentials $E\left[V_H^i\right]$ and the 3D mesh of the user's heart

6. The system of claim 5, where the visualization means are selected from a virtual reality viewer (10) and a holographic display (11).

**Patentansprüche**

1. Verfahren zur dreidimensionalen, stereoskopischen, holographischen 360°-Visualisierung der elektrischen Signale des Herzens, wobei das Verfahren Folgendes umfasst:

• Vorverarbeiten (20-25) von Signalen, die aus Potentialen "$V_T$" des Torsos eines Benutzers gebildet werden, wobei die Potentiale "$V_T$" durch eine textile Vorrichtung mit Elektroden gemessen werden;
• Berechnen (26-28) einer Transfermatrix "$A_{TH}$" zwischen einem 3D-Netz des Herzens des Benutzers und einem 3D-Netz des Torsos des Benutzers: $V_T = A_{TH} V_H$;
• Schätzen (29) der Potentiale "$V_H$" des Herzens des Benutzers aus dem 3D-Netz des Herzens des Benutzers und der Potentiale "$V_T$" des Torsos des Benutzers, wobei die geschätzten Herzpotentiale als $E\left[V_H^i\right]$ bezeichnet werden, wofür ein Wert des Parameters "$\lambda$" berechnet wird, der folgenden Ausdruck minimiert:

$$\epsilon = \left\|A_{TH}E\left[V_H^i\right] - V_T\right\|^2 + \lambda^2\left\|RE\left[V_H^i\right]\right\|^2$$

wobei $R$ eine Identitätsmatrix ist, die als Regularisierungsmatrix bezeichnet wird; so dass $R$ eine Matrix mit $N_H$ Zeilen und $N_H$ Spalten ist;
• Neuberechnen (30) von Position und Ausrichtung des 3D-Netzes des Herzens, wobei die Position und Ausrichtung des 3D-Netzes des Herzens durch den Vektor $\psi = (x_i, y_i, z_i, \theta_i, \rho_i, \phi_i)$ definiert ist;
• Schätzen (30) von Torso-Potentialen: $E\left[V_H^i\right] = A_{TH}E\left[V_H^i\right]$ durch Berechnen des Wertes $\psi$, der den

folgenden Ausdruck minimiert:

$$argmin_\psi \left\| AE\left[V_H^i\right] - V_T \right\|^2 + \lambda^2 \left\| RE\left[V_H^i\right] \right\|^2;$$

- Wiederholen (30) der vorhergehenden Schritte bis $\|E[V_T] - V_T\| < \delta$, wobei $\delta$ eine Toleranz mit einem vordefinierten Wert ist;
- Bereitstellen (31) einer dreidimensionalen, stereoskopischen, holographischen 360°-Visualisierung unter Verwendung der 3D-Visualisierungsmittel, der geschätzten Herzpotentiale $E\left[V_H^i\right]$ und des 3D-Netzes des Herzens des Benutzers.

2. Verfahren nach Anspruch 1, **dadurch gekennzeichnet, dass** der Schritt des Berechnens einer Transfermatrix "$A_{TH}$" zwischen einem 3D-Netz des Herzens des Benutzers und einem 3D-Netz des Torsos des Benutzers des Weiteren umfasst:

- Auswählen (26) des 3D-Netzes des Herzens des Benutzers aus einem Standard-3D-Netz und einem 3D-Netz des Benutzers aus Bildern, die aus Computertomographie- und Magnetresonanzbildern ausgewählt sind;
- Scannen (26) einer Oberfläche des Torsos des Benutzers unter Verwendung einer Vorrichtung, die zum Erzeugen von 3D-Netzen konfiguriert ist.

3. Verfahren nach Anspruch 1, **dadurch gekennzeichnet, dass** der Schritt des Berechnens einer Transfermatrix "$A_{TH}$" des Weiteren die folgenden Teilschritte umfasst:

- Anwenden der zweiten Green'schen Identität, die die Berechnung von elektrischen Potentialen im Inneren eines Volumens "$\theta$" mit der Berechnung auf seiner Oberfläche "S" auf die 3D-Netze des Torsos und des Herzens in Beziehung setzt, wodurch die folgende Gleichung entsteht:

$$\int_S \left( V\nabla\frac{1}{r} - \frac{1}{r}\nabla V \right) \cdot \boldsymbol{n}\,dS = \int_\Theta \left( V\nabla^2\left(\frac{1}{r}\right) - \frac{1}{r}\nabla^2 V \right) d\Theta, \text{ (E2)}$$

wobei $V$ das elektrische Potential und $r$ die Distanz von einem Beobachtungspunkt innerhalb des Volumens zwischen der Oberfläche des Torsos und der Oberfläche des Herzens ist; der Vektor $n$ die Normale zu dem Oberflächenelement $dS$ ist und $\nabla$ der Gradientenoperator ist;
- Anwenden der folgenden Randbedingungen auf die zweite Green'sche Identität:

  ○ C1: $V = V_T$, wenn sich der Beobachtungspunkt auf dem Torso befindet, und $V = V_H$, wenn sich der Beobachtungspunkt auf der Oberfläche des Herzens befindet;
  ○ C2: $\nabla^2 V = 0$;
  ○ C3: $\nabla V_T \cdot \boldsymbol{n} = 0$.

4. Verfahren nach Anspruch 1, **dadurch gekennzeichnet, dass** der Schritt des Vorverarbeitens der Potentiale "$V_T$" des Torsos eines Benutzers die folgenden Teilschritte umfasst:

- Identifizieren und Eliminieren (21) von Ausreißern der durch die Potentiale "$V_T$" gebildeten Signale durch Berücksichtigen der Potentiale "$V_T$", die einen bestimmten Schwellenwert nicht um eine bestimmte Anzahl von Abweichungen überschreiten;
- Anwenden (22) einer "Wellentransformation" zur Zerlegung der aus den Potentialen "$V_T$" gebildeten Signale auf verschiedenen Auflösungsebenen;
- Eliminieren (22) der Wellenkoeffizienten, die Rauschfrequenzen zugehörig sind;
- Rekonstruieren (22) der durch die Potentiale "$V_T$" gebildeten Signale unter Verwendung nur der verbleibenden Koeffizienten;
- Korrigieren (24) des linearen Trends der durch die Potentiale "$V_T$" gebildeten Signale durch Erfassen (23) der Intervalle der Spitzen "R" im resultierenden Elektrokardiogramm "EKG", wobei die Spitzen "R" die lokalen Maxima des QRS-Komplexes sind, die ihrerseits durch die Neigung des absoluten Gradienten des EKG-Signals erfasst werden;
- Auswählen (25) mindestens der zehn besten Sekunden der durch die Potentiale "$V_T$" gebildeten Signale unter Verwendung einer Evaluierung der Qualität jedes Signals mittels eines relativen Indexes.

5. System zur dreidimensionalen, stereoskopischen, holographischen 360°-Visualisierung der elektrischen Signale des Herzens, wobei System Folgendes umfasst:

- eine textile Vorrichtung (2) mit Elektroden (2'), die an den Torso (3) eines Benutzers anpassbar ist, wobei die textile Vorrichtung dazu konfiguriert ist, Potentiale "$V_T$" des Torsos des Benutzers mittels der Elektroden der textilen Vorrichtung zu messen;
- eine 3D-Vernetzungsvorrichtung (6), die dazu konfiguriert ist, ein 3D-Netz des Torsos (3) des Benutzers zu erstellen;
- einen Prozessor (4, 4'), der das 3D-Netz und die Potentiale des Torsos des Benutzers sowie ein 3D-Netz eines Herzens empfängt;
- 3D-Visualisierungsmittel (10, 11);

wobei der Prozessor (4, 4') zu Folgendem konfiguriert ist:

- Vorverarbeiten der Potentiale des Torsos des Benutzers: "$V_T$";
- Berechnen einer Transfermatrix "$A_{TH}$" zwischen dem 3D-Netz des Herzens und einem 3D-Netz des Torsos: $V_T = A_{TH} V_H$;
- Schätzen $E[V_H^i]$ der Potentiale "$V_H$" des Herzens des Benutzers aus dem 3D-Netz und der Potentiale "$V_T$" des Torsos des Benutzers, wobei die geschätzten Herzpotentiale als $E[V_H^i]$| bezeichnet werden, für die ein Parameter "$\lambda$" gesucht wird, der folgenden Ausdruck minimiert:

$$\epsilon = \left\| A_{TH} E[V_H^i] - V_T \right\|^2 + \lambda^2 \left\| R E[V_H^i] \right\|^2$$

wobei R eine Identitätsmatrix ist, die als Regularisierungsmatrix bezeichnet wird; so dass R eine Matrix mit $N_H$ Zeilen und $N_H$ Spalten ist;
- Neuberechnen von Position und Ausrichtung des 3D-Netzes des Herzens, wobei die Position und Ausrichtung des 3D-Netzes des Herzens durch den Vektor $\psi = (x_i, y_i, z_i, \theta_i, \rho_i, \phi_i)$ definiert ist;
- Schätzen von Torso-Potentialen: $E[V_H^i] = A_{TH} E[V_H^i]$ durch Berechnen des Wertes $\psi$, der den folgenden Ausdruck minimiert:

$$argmin_\psi \left\| A E[V_H^i] - V_T \right\|^2 + \lambda^2 \left\| R E[V_H^i] \right\|^2;$$

- Wiederholen der obigen Schritte, bis $\|E[V_T] - V_T\| < \delta$, wobei $\delta$ eine Toleranz ist, die empirisch im Voraus bestimmt wird und bestimmt, dass der Algorithmus konvergiert hat, d. h. $\delta$ eine Toleranz mit einem vordefinierten Wert ist;
- Bereitstellen einer dreidimensionalen, stereoskopischen, holographischen 360°-Visualisierung unter Verwendung der 3D-Visualisierungsmittel, der geschätzten Herzpotentiale $E[V_H^i]$ und des 3D-Netzes des Herzens des Benutzers.

6. System nach Anspruch 5, wobei die Visualisierungsmittel aus einem Virtual-Reality-Viewer (10) und einem holografischen Display (11) ausgewählt sind.

**Revendications**

1. Procédé de visualisation holographique stéréoscopique tridimensionnelle à 360° pour les signaux électriques du cœur, ledit procédé comprend :

- le prétraitement (20-25) de signaux formés par des potentiels « $V_T$ » du torse d'un utilisateur ; les potentiels « $V_T$ » étant mesurés par un dispositif textile doté d'électrodes ;
- le calcul (26-28) d'une matrice de transfert « $A_{TH}$ » entre un maillage 3D du cœur de l'utilisateur et un maillage 3D du torse de l'utilisateur : $V_T = A_{TH} V_H$ ;
- l'estimation (29) de potentiels « $V_H$ » du cœur de l'utilisateur à partir du maillage 3D du cœur de l'utilisateur et des potentiels « $V_T$ » du torse de l'utilisateur, les potentiels du cœur estimés étant appelés $E\left[V_H^i\right]$, pour lesquels une valeur du paramètre « $\lambda$ » est calculée, qui minimise l'expression :

$$\epsilon = \left\|A_{TH}E\left[V_H^i\right] - V_T\right\|^2 + \lambda^2\left\|RE\left[V_H^i\right]\right\|^2$$

R étant une matrice unité, qui est appelée une matrice de régularisation ; de sorte que R soit une matrice de $N_H$ rangées et $N_H$ colonnes ;
• le recalcul (30) de la position et de l'orientation du maillage 3D du cœur, la position et l'orientation du maillage 3D du cœur étant définies par le vecteur $\psi = (x_i, y_i, y_i, \theta_i, \rho_i, \phi_i)$ ;

• l'estimation (30) de potentiels du torse : $E\left[V_T^i\right] = A_{TH}E\left[V_H^i\right]$ par le calcul de la valeur de $\psi$ qui minimise l'expression suivante :

$$argmin_\psi\left\|\boldsymbol{A}E\left[V_H^i\right] - V_T\right\|^2 + \lambda^2\left\|\boldsymbol{R}E\left[V_H^i\right]\right\|^2 \; ;$$

• la répétition (30) des étapes précédentes jusqu'à ce que $\|E[V_T] - V_T\| < \delta$, $\delta$ étant une tolérance ayant une valeur prédéfinie ;
• la fourniture (31) d'une représentation holographique stéréoscopique 3D à 360° à l'aide des moyens de visualisation 3D, des potentiels du cœur estimés $E\left[V_H^i\right]$ et du maillage 3D du cœur de l'utilisateur.

2. Procédé selon la revendication 1, **caractérisé en ce que** l'étape de calcul d'une matrice de transfert « $A_{TH}$ » entre un maillage 3D du cœur de l'utilisateur et un maillage 3D du torse de l'utilisateur comprend en outre :

• la sélection (26) du maillage 3D du cœur de l'utilisateur parmi un maillage 3D standard et un maillage 3D de l'utilisateur à partir d'images sélectionnées parmi des images de tomodensitométrie et des images de résonance magnétique ;
• le balayage (26) d'une surface du torse de l'utilisateur à l'aide d'un dispositif configuré pour la génération de maillages 3D.

3. Procédé selon la revendication 1, **caractérisé en ce que** l'étape de calcul d'une matrice de transfert « $A_{TH}$ » comprend en outre les sous-étapes suivantes :

• l'application de la deuxième identité de Green qui associe le calcul de potentiels électriques à l'intérieur d'un volume « $\theta$ » avec ledit calcul sur sa surface « S » aux maillages 3D du torse et du cœur, ce qui génère l'équation suivante :

$$\int_S \left(V\nabla\frac{1}{r} - \frac{1}{r}\nabla V\right)\cdot\boldsymbol{n}dS = \int_\Theta \left(V\nabla^2\left(\frac{1}{r}\right) - \frac{1}{r}\nabla^2 V\right)d\Theta \; , \; (E2)$$

V étant le potentiel électrique et r la distance par rapport à un point d'observation à l'intérieur du volume entre la surface du torse et la surface du cœur ; le vecteur n étant la normale à l'élément de surface dS et V étant l'opérateur de gradient ;
• l'application des conditions aux limites suivantes à la deuxième identité de Green :

  ◦ C1 : $V = V_T$ lorsque le point d'observation est sur le torse et $V = V_H$ lorsque le point d'observation est sur la surface du cœur ;
  ◦ C2 : $\nabla^2 V = 0$ ;
  ◦ C3: $\nabla V_T \cdot n = 0$.

4. Procédé selon la revendication 1, **caractérisé en ce que** l'étape de prétraitement de potentiels « $V_T$ » du torse d'un utilisateur comprend les sous-étapes suivantes :

• l'identification et l'élimination (21) de signaux aberrants parmi les signaux formés par les potentiels « $V_T$ » en considérant les potentiels « $V_T$ » qui ne dépassent pas un certain seuil d'un nombre donné d'écarts ;

- l'application (22) d'une transformée en « ondelettes » pour décomposer les signaux formés par les potentiels « $V_T$ » à différents niveaux de résolution ;
- l'élimination (22) des coefficients d'ondelettes associés à des fréquences de bruit ;
- la reconstruction (22) des signaux formés par les potentiels « $V_T$ » uniquement à l'aide des coefficients restants ;
- la correction (24) de la tendance linéaire des signaux formés par les potentiels « $V_T$ » par la détection (23) des intervalles des pics « R » dans l'électrocardiogramme « ECG » résultant, les pics « R » étant les maximums locaux du complexe QRS, qui à leur tour sont détectés par le biais de l'inclinaison du gradient absolu du signal d'ECG ;
- la sélection (25) d'au moins les dix meilleures secondes des signaux formés par les potentiels « $V_T$ », à l'aide d'une évaluation de la qualité de chaque signal au moyen d'un indice associé.

5. Système de visualisation holographique stéréoscopique tridimensionnelle à 360° pour les signaux électriques du cœur, ledit système comprenant :

   - un dispositif textile (2) doté d'électrodes (2'), qui est ajustable sur le torse (3) d'un utilisateur, le dispositif textile étant configuré pour la mesure de potentiels « $V_T$ » du torse de l'utilisateur au moyen des électrodes du dispositif textile ;
   - un dispositif de maillage 3D (6), qui est configuré pour la création d'un maillage 3D du torse (3) de l'utilisateur ;
   - un processeur (4, 4') qui reçoit le maillage 3D et des potentiels du torse de l'utilisateur, et un maillage 3D d'un cœur ;
   - des moyens de visualisation en 3D (10, 11) ;

   le processeur (4, 4') étant configuré pour :

   - prétraiter les potentiels du torse de l'utilisateur : « $V_T$ » ;
   - calculer une matrice de transfert « $A_{TH}$ » entre le maillage 3D du cœur et le maillage 3D du torse : $V_T = A_{TH} V_H$ ;
   - estimer $E\left[V_H^i\right]$ potentiels « $V_H$ » du cœur de l'utilisateur à partir du maillage 3D et des potentiels « $V_T$ » du torse de l'utilisateur, les potentiels du cœur estimés étant appelés $E\left[V_H^i\right]$, pour lesquels un paramètre « $\lambda$ » est recherché, qui minimise l'expression :

$$\epsilon = \left\|A_{TH}E\left[V_H^i\right] - V_T\right\|^2 + \lambda^2\left\|RE\left[V_H^i\right]\right\|^2$$

   R étant une matrice unité, qui est appelée une matrice de régularisation ; de sorte que R soit une matrice de $N_H$ rangées et $N_H$ colonnes ;
   - recalculer la position et l'orientation du maillage 3D du cœur, la position et l'orientation du maillage 3D du cœur étant définies par le vecteur $\psi = (x_i, y_i, y_i, \theta_i, \rho_i, \phi_i)$
   - estimer des potentiels du torse : $E\left[V_T^i\right] = A_{TH}E\left[V_H^i\right]$ par le calcul de la valeur de $\psi$ qui minimise l'expression suivante :

$$argmin_\psi\left\|AE\left[V_H^i\right] - E\left[V_T^i\right]\right\|^2 + \lambda^2\left\|RE\left[V_H^i\right]\right\|^2$$

   - répéter les étapes ci-dessus jusqu'à ce que $\|E[V_T] - V_T\| < \delta$, $\delta$ étant la tolérance qui est déterminée empiriquement a priori et déterminant que l'algorithme a convergé, autrement dit, $\delta$ étant une tolérance ayant une valeur prédéfinie ;
   - fournir une représentation holographique stéréoscopique 3D à 360° à l'aide des moyens de visualisation 3D, des potentiels du cœur estimés $E\left[V_H^i\right]$ et du maillage 3D du cœur de l'utilisateur.

6. Système selon la revendication 5, les moyens de visualisation étant sélectionnés parmi une visionneuse de réalité virtuelle (10) et un afficheur holographique (11).

**FIG. 1**

FIG. 2

# EP 4 501 233 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SALINET JOÃO et al.** Electrocardiographic Imaging for Atrial Fibrillation: A Perspective From Computer Models and Animal Experiments to Clinical Value. *FRONTIERS IN PHYSIOLOGY*, 30 April 2021, vol. 12